# EUROPÄISCHE PATENTSCHRIFT

(11) **EP 0 674 525 B1**
(45) Veröffentlichungstag und Bekanntmachung des Hinweises auf die Patenterteilung: **25.07.2001**
(21) Anmeldenummer: 94903779.0
(22) Anmeldetag: 15.12.1993
(51) Int. Cl.: A61K 38/00, A61K 9/14, A61K 9/19, A61K 47/18

(54) **LAGERSTABILE WÄSSRIGE PHARMAZEUTISCHE ZUBEREITUNGEN VON G-CSF**
AQUEOUS PHARMACEUTICAL PREPARATIONS OF G-CSF WITH A LONG SHELF LIFE
PREPARATIONS PHARMACEUTIQUES AQUEUSES DE G-CSF STABLES AU STOCKAGE

(30) Priorität: 18.12.1992 DE 4242919
(43) Veröffentlichungstag der Anmeldung: 04.10.1995
(73) Patentinhaber: Roche Diagnostics GmbH, 68305 Mannheim (DE)
(72) Erfinder: MICHAELIS, Uwe, D-82362 Weilheim (DE); RUDOLPH, Rainer, D-82362 Weilheim (DE); WINTER, Gerhard, D-69221 Dossenheim (DE); WOOG, Heinrich, D-69514 Laudenbach (DE)
(86) Internationale Anmeldenummer: EP9303544
(87) Internationale Veröffentlichungsnummer: WO9414466

(56) Entgegenhaltungen:
- EP-A- 0 306 824
- EP-A- 0 373 679
- EP-A- 0 528 314
- DE-A- 3 723 781
- PATENT ABSTRACTS OF JAPAN vol. 016, no. 289 (C-0956)26. Juni 1992 & JP,A,04 077 434 (KANJI TANAKA) 11. März 1992

## Beschreibung

Gegenstand der vorliegenden Erfindung sind lagerstabile wässrige pharmazeutische Zubereitungen von G-CSF, die zur Stabilisierung eine Tensidmenge, die kleiner der eingesetzten Menge an G-CSF ist, sowie mindestens einen Puffer ausgewählt aus der Gruppe Essigsäure, Milchsäure. Citronensäure, Maleinsäure, Phosphorsäure und deren Salze oder Arginin und seiner Salze in einer Konzentration von jeweils bis zu 100 mMol/l enthalten.

Verschiedene pharmazeutische Zubereitungen. die G-CSF (Granulozyten- Kolonie stimmulierender Faktor) enthalten, sind bereits aus dem Stand der Technik bekannt.

In DE 37 23 781 (GB 2.193.631) wird ein G-CSF-haltiges Arzneimittel beschrieben. das zur Stabilisierung von G-CSF mindestens ein pharmazeutisch verträgliches grengfichenaktives Miflel, Saccharid. Protein oder eine nochmolekulare Verbindung enthält. Es werden dort Zubereitungen vorgeschlagen, die Humanserumalbumin als stabilisierendes Mittel enthalten. Als vorteilhaft werden insbesondere Zubereitungen genannt, die oberflächenaktive Mittel in Gewichtsanteilen enthalten. die dem 1 - 10 000fachen der eingesetzten G-CSF-Menge entsprechen.

In EP 0 373 679 werden stabilisierte Zubereitungen von G-CSF beschrieben, die sich im wesentlichen durch einen sauren pH-Wert der Lösung auszeichnen, wobei die Lösungen eine möglichst geringe Leitfähigkeit aufweisen. Die Lösungen weisen einen pH-Wert von 3 - 3.7 auf, falls die Lösungen weitere pharmazeutische Hifsstoffe, wie beispielsweise Puffer oder Mannitol enthalten. Für den Fall, daß keine Puffersubstanzen in der Arzneiform vorhanden sind, werden pH-Bereiche von 2.75-4 als vorteilhaft beschrieben.

In EP 0 306 824 werden stabilisierte Lyophilisate von Humanprotein-Präparaten beschrieben, bei denen die Stabilisierung durch Zusatz eines Gemisches von Harnstoff. Aminosäuren und Detergens erfolgt.

In der früheren PCT-Patentanmeldung PCT/EP92/01823 wird ein Verfahren zur Herstellung von G-CSF-haltigen, gut verträglichen Arzneimitteln für Infusions- und Injektionszwecke beschrieben. Die flüssigen Darreichungsformen zeichnen sich insbesondere durch eine geringe Titrationsacidität und geringe Pufferkapazität aus. Die pH-Werte der beschriebenen G-CSF- haltigen Infusions- und Injektionslösungan liegen im sauren Bereich von 3,8 - 4,5.

Verfahren zur Herstellung von G-CSF-haltigen flüssigen Arzneiformen, die zusätzlich Konservierungsmittel enthalten. sind bekannt aus PCT/EP92/01822. Die pH-Werte der pharmazeutischen Lösungen liegen im sauren Bereich von 2,5 - 4.5. Die Stabilisierung von G-CSF wird dort im wesentlichen durch die Einstellung des für G-CSF gunstigen sauren pH-Wertes und durch Zugabe einer Mischung von verschiedenen Aminosäuren erreicht.

In EP 0 528 314 A1 wird ein Verfahren zur Herstellung von humanprotein-haltigen Arzneimitteln zur Anwendung als Injektions- oder Infusionslösungen beschrieben, wobei die Lösungen gut verträglich sind und einen pH-Wert von 2-7,4 und eine Pufferkapazität von bis zu 10 mVal/l aufweisen.

Die bisher bekannten Arzneiformen tür G-CSF besitzen jedoch einige Nachteile. Die in DE 37 23 781 beschriebenen Zubereitungen enthalten pharmazeutische Zusatz- oder Hilfsstoffe, die aus medizinischer Sicht nicht ohne weiteres als unbedenklich einzustufen sind. Polymere und Proteine besitzen im Hinblick auf ihre Eignung als pharmazeutische Zusatzstoffe aufgrund ihrer Herkunft und ihrer physikalischchemischen Eigenschaften ein gewisses Risikopotential. Proteine menschlichen oder tierischen Ursprungs sowie aus Zellkulturen gewonnene Proteine tragen ein potentielles Restrisiko viraler Verunreinigungen. Auch andere, analytisch schwer nachweisbare proteinartige Verunreinigungen können wegen ihrer antigenen Eigenschaften immunologische Reaktionen beim Menschen hervorrufen. Proteine tierischen Ursprungs können darüber hinaus generell aufgrund ihrer species-spezifischen Eigenschaften immunologische Reaktionen beim Menschen auslösen. Auch Langzeitreaktionen nach späterer Reapplikation derartiger Proteine sind möglich.

Der Zusatz von hochmolekularen Verbindungen kann ebenfalls problematisch sein. Polymere sind aufgrund ihrer großen Molekülmasse im Körper akkumulierbar und können somit, falls kein Bioabbau erfolgt, über lange Zeit im Körper verbleiben. Dies ist besonders bei subkutaner Applikation zu befürchten, da der Abtransport und die Verteilung durch den Blutstrom gegenüber intravenöser Gabe stark verlangsamt erfolgt. In Abhängigkeit von der Molmasse können Polymere auch antigene Eigenschaften aufweisen. Auch ist die Reinheit von Polymeren aufgrund der Zur Herstellung verwendeten Katalysatoren oder des Vorhandenseins von Monomeren und anderen Polymer-Bruchstücken schwierig zu gewährleisten. Der Einsatz von Polymeren bei flüssigen pharmazeutischen Darreichungsformen, insbesondere bei subkutan applizierbaren Arzneiformen ist somit, wenn möglich, zu vermeiden.

Die in DE 37 23 781 beschriebenen Tensidmengen sind aus medizinischer Sicht ebenfalls als problematisch anzusehen. Dort werden Tensidkonzentrationen als vorteilhaft beschrieben, die bezüglich der Gewichtsanteile von G-CSF 110000 Gewichtsanteile eines oberflächenaktiven Mittels enthalten. Betrachtet man andererseits die für den klinischen Gebrauch bevorzugten Anwendungskonzentrationen von G-CSF von 0,05 - 1,5mg/ml in den fertigen Arzneiformen, so ergeben sich entsprechend hohe Tensidkonzentrationen. Diese sind aus medizinischer Sicht zu vermeiden, da sie lokale Irritationen auslösen können.

Die oben beschriebenen Formulierungen haben den Nachteil, daß sie aufgrund des angewandten niedrigen pHWertes insbesondere bei subkutaner Anwendung zu lokalen Unverträglichkeiten beim Patienten führen. Das erhaltene Produkt kann bei empfindlichen Patienten zu Schmerzen und lokaler Gewebereizung führen, da der im Gewebe physiologisch vorliegende Bereich von pH 7,0 - 7,5 nicht eingehalten wird.

Aus der Literatur ist ferner bekannt, daß insbesondere nicht glykosilierte Formen von G-CSF gegenüber glykosiliertem GCSF, das aus CHO-Zellen gewonnen wird, besonders instabil sind (J. Biol. Chem. 1990, 265, 11432). Die Stabilisierung von nicht glykosilierten Formen von G-CSF ist deshalb besonders schwierig und bedarf speziell ausgewählter Maßnahmen, um dieses Molekül in einer stabilen Arzneiform zu formulieren.

Als wesentlicher Nachteil von flüssigen G-CSF-haltigen Arzneiformen wurde darüberhinaus jedoch festgestellt, daß sie vor allem bei längerer Lagerung oder im Verlauf des Transportes auf den Vertriebswegen relativ leicht zur Bildung von Trübungen neigen. Insbesondere hat sich gezeigt, daß die applizierbaren Lösungen eine erhöhte Empfindlichkeit gegenüber mechanischem Streß aufweisen. Mechanische Belastungen, wie beispielsweise Schütteln der Lösung, kann während des Transportes der flüssigen Arzneiformen in vielfältiger und unkontrollierbarer Weise auf sie einwirken. Auch bei der Anwendung durch den Arzt, medizinisches Personal oder den Patienten können falsche Handhabung und damit möglicherweise mechanische Belastungen der in Ampullen oder Vials abgefüllten pharmazeutischen Lösung nicht mit Sicherheit ausgeschlossen werden. Dementsprechend ist die Robustheit gegenüber mechanischem Streß ein Qualitätskriterium für Proteinarzneimittel. Maßnahmen zur Aufrechterhaltung der Stabilität von G- CSF gegenüber mechanischer Belastung wurden In der Literatur bislang nicht beschrieben. Nach üblichen Verfahren hergestellte flüssige Zubereitungen von G-CSF weisen zwar eine ausreichende Stabilltät selbst bei Lagerung unter erhöhten Temperaturen auf. jedoch ist die Stabilität derartiger Rezepturen gegenüber mechanischer Belastung nicht in allen Fällen befriedigend. Insbesondere treten häufig sichtbare Trübungen der Arzneilösungen auf, die durch die Bildung von Dimeren oder Aggregaten von G-CSF hervorgerufen werden. Derartige Veränderungen der flüssigen Arzneiformen wirken sich vor allem negativ auf die Menge des in der einzeinen Darreichungsform enthaltenen aktiven Wirkstoffes aus und sind aus medizinischer Sicht möglichst zu vermeiden.

Aufgabe der vorliegenden Erfindung war es, eine stabile flüssige Arzneiform für G-CSF zur Verfügung zu stellen, die eine ordnungsgemäße Anwendung von G-CSF als Arzneimittel ermöglicht, und die oben beschriebenen Nachteile der bisher bekannten Arzneiformen nicht aufweist. Die pharmazeutische Zubereitung sollte insbesondere langfristig lagerstabil, stabil gegenüber mechanischer Belastung, physiologisch gut verträglich, einfach handhabbar und exakt dosierbar sein. Sie sollte insbesondere einen physiologisch verträglichen pH-Wert aufweisen.

Überraschenderweise wurde gefunden, daß man im Sinne der vorliegenden Erfindung stabile wässrige pharmazeutische Arznerformen herstellen kann, wenn man als Zusatstoffe eine Tensidmenge, die kleiner der eingesetzten Menge an G-CSF ist, sowie eine Puffersubstanz ausgewählt aus der Gruppe Acetat, Lactat, Citrat. Maleat oder Phosphat einsetzt. Die Puffer werden vorteilhaft in einer solchen Menge eingesetzt, daß die Konzentration der jeweils ausgewählten Puffersubstanz in der fertigen applizierbaren Arzneiform, die in den Handel kommt, von 2 bis zu 100 mMol/l beträgt. Die so hergestellten Lösungen sind überraschenderweise weitgehend stabil gegenüber mechanischer Belastung. Sie haben außerdem den Vorteil. daß durch die getroffene Auswahl der Puffersubstanzen Lösungen zur Verfügung gestellt werden können, die einen vorteilhaften pH-Wert von 4 - 5 oder 7 - 8 aufweisen, während die aus dem Stand der Technik bekannten Lösungen aus Stabilitätsgründen des Proteins vorzugsweise Lösungen mit einem pH-Wert von 2,5 - 3,5 erforderlich machten.

Die erfindungsgemäß hergestellten Zubereitungen besitzen außerdem den Vorteil, daß sie frei von proteinartigen oder polymeren Hilfsstoffen sind, deren Verwendung aus medizinischer Sicht problematisch sein kann. Aufgrund der Tatsache, daß nunmehr flüssige G-CSF-haltige Arzneiformen mit einem pH- Wert von 7 - 8, d.h. mit einem pH-Wert in der Nähe des pH-Wertes des Blutes (pH 7,2 - 7,4), hergestellt werden können, besitzen sie ferner den Vorteil, gut verträglich und weitgehend schmerzfrei applizierbar zu sein.

Vorteilhaft ist ferner, daß aufgrund der getroffenen Auswahl der Hilfsstoffe die bisher benötigten relativ hohen Tensidmengen in den flüssigen Arzneiformen nicht mehr erforderlich sind. Vielmehr sind niedrige Tensidmengen von 0,5 mg/ml oder weniger, vorzugsweise von 0,01 - 0,1 mg/ml, ausreichend zur Stabilisierung von G-CSF. Vorteilhaft können Tensidkonzentrationen (mg/ml) verwendet werden, die kleiner als oder maximal gleich der eingesetzten Proteinmenge an G-CSF pro Volumeneinheit (mg/ml) sind. Dies ist vor allem bei solchen flüssigen Arzneiformen von Vorteil, die für die subkutane Anwendung von G-CSF bestimmt sind. Außerdem werden durch die erfindungsgemäßen Maßnahmen insbesondere die labilen, unglykosilierten G-CSF-Moleküle für pharmazeutische Zubereitungen ausreichend stabilisiert. Durch die gezielt getroffene Auswahl der Hilfsstoffe werden insgesamt sehr gut verträgliche G-CSF-haltige Arzneiformen zur Verfügung gestellt, die bezüglich der Proteinstabilität qualitativ hochwertige Zubereitungen darstellen.

Die erfindungsgemäßen G-CSF-haltigen Arzneiformen enthalten den Wirkstoff in einer zur Erzielung eines therapeutischen Effektes ausreichenden Menge. In der Regel werden Wirkstoffkonzentrationen von 0,01 - 5 mg/ml verwendet, vorzugsweise 0,1 -1,5 mg/ml.

Erfindungsgemäß werden als pharmazeutische Hilfsstoffe und Puffersubstanzen Essigsäure, Zitronensäure, Milchsäure, Maleinsäure und Phophorsäure oder deren physiologisch verträglichen Salze eingesetzt. Bei der Herstellung der Hilfsstofflösung können diese Puffersubstanzen entweder in Form der entsprechenden freien Säure oder in Form der Alkali-, Erdalkali- oder Ammoniumsalze vorgegeben werden. Außerdem kann die Lösung weitere pharmazeutisch übliche Hilfsstoffe enthalten. Die Reihenfolge der Zugabe der verschiedenen Hilfsstoffe oder des Wirkstoffes bei der Herstellung der flüssigen Arzneiformen ist weitgehend unabhängig hinsichtlich der erfindungsgemäß gefundenen lagerstabilisierenden Wirkung und liegt im Ermessen des Fachmannes. Der gewünschte pH-Wert der Lösung wird durch Zugabe von Basen, wie beispielsweise von Alkalihydroxiden, Erdalkalihydroxiden oder Ammoniumhydroxid eingestellt. Vorzugsweise wird hierzu Natriumhydroxid verwendet. Die Einstellung des gewünschten pH-Wertes ist prinzipiell durch Zugabe von basischen Lösungen möglich. In diesem Sinne kommen allgemein Salze von starken Basen mit schwachen Säuren in Frage, wie z. B. Natriumacetat, Natriumcitrat, Di-Natrium- bzw. Di-Kalium-hydrogenphosphat oder Natriumcarbonat. Für den Fall, daß die pharmazeutische Hilfsstofflösung einen basischen pH-Wert aufweist, erfolgt die Einstellung durch Titration mit Säure, bis der gewünschte pH-Bereich von 4 - 5 oder 7 - 8 erreicht ist. Als Säuren kommen physiologisch verträgliche anorganische oder organische Säuren in Frage, wie beispielsweise Salzsäure, Phosphorsäure, Essigsäure, Zitronensäure oder allgemein übliche Lösungen von Substanzen, die einen sauren pH-Wert besitzen. Bevorzugte Substanzen sind in diesem Sinne Salze von starken Säuren mit schwachen Basen, wie z.B. Natriumdihydrogenphosphat oder Kaliumdihydrogenphosphat.

Auch Arginin und seine Salze werden erfindungsgemäß als pharmazeutische Hilfsstoffe und Puffersubstanzen eingesetzt.

Die Konzentrationen der Puffersubstanzen Essigsäure, Zitronensäure, Milchsäure, Maleinsäure oder Phosphorsäure in der fertig applizierbaren flüssigen Arzneiform betragen jeweils etwa 2 - 100 mMol/l. Aufgrund der Tatsache, daß die vorgenannten Säuren bei der Herstellung der pharmazeutischen Hilfsstofflösung häufig in Form ihrer Salze eingesetzt werden, und seltener in Form der freien Säure, wird im folgenden der Einfachheit halber jeweils Bezug genommen auf die Anionen-Konzentrationen dieser Säuren, also beispielsweise Acetat, Citrat, Lactat, Maleat oder Phosphat. Die Gesamtkonzentrationen an Puffersubstanzen sollte einen Wert von 100 mMol/l, vorzugsweise 80 mMol/l, nicht übersteigen. Bevorzugt beträgt die Gesamtkonzentration der Puffersubstanzen etwa 5 - 40 mMol/l.

Es hat sich gezeigt, daß bestimmte pH-Bereiche der flüssigen Arzneiform in Kombination mit bestimmten Puffersubstanzen besonders stabile Lösungen ergeben. Bei Verwendung eines Acetat- oder Lactatpuffers in einer Konzentration von 5 - 40 mMol/l und Einstellung des pH-Wertes der Lösung auf einen Bereich von etwa 2 - 5 wird eine gegenüber mechanischer Belastung besonders stabile Arzneiform erreicht. Bevorzugt werden folgende Pufferkonzentrationen und pH-Werte verwendet: 5 - 80 mMol/l, insbesondere 10 - 30 mMol/l, Acetat oder Lactat und pH 3,5 - 5.

Citrat wird in einer Konzentration von 5 - 40mMol/l, vorzugsweise 5 - 20 mMol/l, eingesetzt. Bevorzugt wird eine Kombination der Puffersubstanzen Citrat und Phosphat verwendet, wobei die Gesamtkonzentration der Puffersubstanzen 10 - 40 mMol/l, vorzugsweise 15 - 30mMol/l beträgt. Die pH-Werte der flüssigen Arzneiform liegen hierbei vorteilhafterweise im Bereich von etwa 2,5 - 3.5 oder 7 - 7,5.

Maleat wird vorzugsweise in einer Konzentration von 5 - 40 mMol/l eingesetzt. Die pH-Werte der flüssigen Arzneiform liegen hierbei vorteilhafterweise im Bereich von etwa 2,5 - 3,5 oder 7 - 7,5.

Phosphat wird in einer Konzentration von 5 - 80 mMol/1. vorzugsweise von 5 - 30 mMol/l, allein oder in Kombination mit einer der anderen Puffersubstanzen verwendet. Die pH-Werte der flüssigen Arzneiform, die allein Phosphatpuffer enthält. liegen vorteilhafterweise im Bereich von etwa 3,5 - 5 oder 7 - 8.

Argininphosphat. Argininchlorid- und Arginincitratpuffer wird ebenfalls in einer Konzentrtion von 2 - 100 mMal/l, vorzugsweise 5 - 80 mMol/l, eingesetzt. Der pH-Wert der flüssigen Zubereitung, die Argininpuffer enthält, liegt bei etwa 7 - 8, vorzugsweise bei 7 - 7,5.

Die erfindungsgemäß beschriebene Auswahl von Puffersystemen bei physiologisch verträglichen pH-Werten und in physiologisch verträglichen Konzentrationen ist auch bei aus Lyophilisaten oder Pulvern durch Wiederauflösen hergestellten Lösungen von G-CSF sinnvoll und vorteilhaft.

Da beim Wiederauflösen von Lyophilisaten mechanische Agitation ausgeübt wird (Schütteln), ist hierbei die gezielte Auswahl bestimmter Puffersysteme und pH-werte wientig. Die Auswahl nicht erfindungsgemäßer Puffersysteme oder pH-Werte kann zur Ausbildung von Aggregationen, Trübungen und somit zu einem qualitativ minderwertigen Produkt führen.

Es bleibt dabei dem Fachmann überiassen, ob die zur Herstellung der erfindungs- gemäßen pH-Werte und Puffersysteme notwendigen Säuren. Salze, Basen im Lyophilisat oder in der zur Auflösung benutzten wäßrigen Lösung oder in beiden Komponenten enthalten sind.

Aus den erfindungsgemäßen wäßrigen Zubereitungen lassen sich durch übliche Lyophilisation Lyophilisate oder z. B, durch Sprühtrocknung Pulver herstellen. Durch Auflösung dieser in Wasser oder wäßrigen Lösungen werden wiederum die erfindungsgemäßen Zubereitungen erhalten. Es hat sich gezeigt, daß wiederaufgelöste Lyophilisate, die Argininpuffer im pH-Bereich von 7 - 7,5 enthalten, mindestens 24 Stunden haltbar sind.

Die mittels der genannten Puffersubstanzen ünd der angegebenen Tensidmenge mögliche Stabilisierung von G-CSF-Molekülen bezieht sich prinzipiell auf alle durch rekombinante Verfahren hergestellte G-CSF-Moleküle und deren Varianten. Der Begriff G-CSF oder G-CSF-Variante gemäß vorliegender Erfindung beinhaltet alle natürlich vorkommenden Varianten von G-CSF. sowie davon abgeleitete durch rekombi- nante DNA-Technologie modifizierte G-CSF-Proteine, insbe- sondere Fusionsproteine, die neben dem G-CSF-Anteil noch andere Proteinsequenzen enthalten. Besonders bevorzugt ist in diesem Sinne ein G-CSF-Mutein mit einem N-terminalen Met-Rest an Position - 1, das zur Expression in prokaryontischen Zellen geeignet ist. Ebenso geeignet ist eine rekombinante methioninfreie G-CSF-Variante. die gemäß PCT/EP91/00192 hergestellt werden kann. Unter dem Begriff "GCSF-Variante" werden solche G-CSF-Moleküle verstanden. bei denen eine oder mehrere Aminosäuren deletiert oder durch andere Aminosäuren ersetzt sein können. wobei die wesentlichen Eigenschaften von G-CSF weitgehend erhalten bleiben. Geeignete G-CSF-Muteine sind beispielsweise in EP 0 456 200 beschrieben.

Zur Prüfung der Stabilität der flüssigen Arzneiformen gegenüber mechanischer Belastung eignet sich insbesondere die Messung der Trübung von G-CSF-haltigen Lösungen. Die Bewertung der Trübung einer Proteinlösung nach Einwirkung von mechanischem Streß ist als einfach auszuführende Prüfmethode besonders wertvoll. Das Auftreten von Trübungen korreliert mit dem Entstehen von Polymeren, Oligomeren und Aggregaten. Die optische Begutachtung erweist sich in einigen Fällen den spezielleren Methoden zur Detektion von Dimeren und Aggregaten (z.B. HPLC-Methoden) überlegen, da große Aggregate bei der Trübungsmethode nicht durch die Probenvorbereitung, wie beispielsweise im Falle der HPLC erforderlich, der Quantifizierung entzogen worden, sondern im Originalgefäß begutachtet und so mit Sicherheit erkannt werden können. Die Quantifizierung von Trübungserscheinungen ist mit handelsüblichen Turbidimetern, Streulichtphotometem, etc. leicht möglich. Die Bewertung derartiger Ergebnisse läßt sich auch in die Anforderungen der Arzneibücher übertragen, die z.B. Trübungseckwerte definieren, unterhalb derer Lösungen als klar bzw. leicht getrübt angesehen werden können.

Zur Herstellung gut verträglicher parenteraler Arzneiformen ist der Zusatz von isotonisierenden Hilfsstoffen zweckmäßig, wenn nicht durch die osmotischen Eigenschaften des Wirkstoffes und der zur Stabilisierung eingesetzten Hilfsstoffe bereits Isotonie erreicht werden kann. Dazu werden vor allem nicht-ionisierte, gut verträgliche Hilfsstoffe, wie z. B. Mannit, Glycerol oder andere Zuckeralkohole eingesetzt.

Im Falle von G-CSF wird vorzugsweise Mannit eingesetzt, ohne daß dieses für die Stabilität von G-CSF wesentlich ist. Die Stabilität des Proteins ist in gleicher Weise auch in Mannit- freien Zubereitungen gegeben, jedoch sind die Lösungen dann aufgrund ihrer mangelnden Isotonie weniger gut verträglich.

Der Zusatz von Salzen ist zur Einstellung der Isotonie nicht vorteilhaft, da hohe Salz- oder lonenkonzentrationen die Aggregatbildung von G-CSF fördern. Vorteilhaft werden deshalb Salze in geringer Menge zugesetzt. Die Pufferkonzentrationen sind so bemessen, daß der pH-stabilisierende Effekt zwar erreicht, die lonenstärke jedoch so gering als möglich gehalten wird. Die Pufferkonzentrationen liegen vorzugsweise im Bereich von bis zu 80mMol/l, besonders bevorzugt weniger als 30 mMol/l.

Außerdem können die fertig applizierbaren Injektionslösungen weitere übliche Hilfs- oder Zusatzstoffe enthalten. Es können Antioxidantien, wie beispielsweise Glutathion, Ascorbinsäure oder ähnliche Substanzen, chaotrope Hilfsstoffe, wie beispielsweise Harnstoff, und Aminosäuren, wie beispielsweise Arginin, Lysin, Ornithin, u.a. zugesetzt werden.

Im folgenden wird die Erfindung anhand von repräsentativen Ausführungsbeispielen näher beschrieben:

### Beispiel 1:

### Allgemeines Verfahren zur Herstellung der flüssigen Arzneiformen

Die in den Beispielen verwendeten Lösungen von G-CSF wurden hergestellt, indem die beschriebenen Hilfsstoffe in Wasser für Injektionszwecke gelöst wurden, GCSF in der angegebenen Menge zugesetzt wurde und, falls notwendig, der pH-Wert mit einer kleinen Menge Pufferkomponente genau auf den Sollwert eingestellt wurde. Die Lösungen wurden dann durch geeignete sterilisierte Membranfilter von 0,2 µm Porenweite filtriert und in Injektionsfläschlein aus Glas der hydrolytischen Klasse I abgefüllt und mit sterilen, teflonisierten Gummistopfen verschlossen. Die Abfüllung erfolgte vorzugsweise unter Stickstoffbegasung.

### Beispiel 2:

### Untersuchungsmethoden zur Stabilitätsbestimmung

Die verschlossenen und verbördelten Flaschen wurden unter Lichtausschluß bei definierten Lagertemperaturen gelagert und danach mit reversed phase HPLC (RP-HPLC), Gelchromatographie oder size exclusion Chromatographie (SEC HPLC) und Western Blot auf Proteinreinheit sowie das Auftreten von Aggregaten und Dimeren hin untersucht. Die angewandten Methoden lassen sich wie folgt beschreiben:
2.1 Reversed phase HPLC
   Die RP-HPLC erfolgte unter Verwendung einer Nucleosil C18-Säule (Fa. Knauer). Die mobile Phase bestand aus 0,12 % (v/v) Trifluoressigsäure (TFA)/Wasser (A) und 0,1 % (v/v) TFA/Acetonitril (B). Die Chromatographie wurde mit einer Flußrate von 0,5 ml/min unter Verwendung eines linearen Gradienten von A nach B durchgeführt.
   Die Injektionsmenge betrug je nach Rezeptur 3 - 6 µg G-CSF. Die Auswertung erfolgte über die Peakfläche unter Verwendung eines externen Standards bei einer Wellenlänge von 214nm.
2.2 Size Exclusion Chromatographie (SEC)
   Für die SE-Chromatographie wurde eine TSK G 2000 SW-Trennsäule (7,5 x 300 mm) verwendet. Die Trennungen erfolgten isokratisch bei Raumtemperatur und einer Flußrate von 0,6ml/min in einem Phosphatpuffer (22,2 mM Na₂HP04; 107,7mM KH₂PO₄; pH 6,2). Die Injektionsmenge betrug 3 - 6 µg G-CSF. Die Auswertung erfolgte über die Peakfläche unter Verwendung eines externen Standards bei einer Detektionswellenlänge von 214nm.
2.3 SDS-Page/Western Blot
   3 µg rhG-CSF werden unter nichtreduzierenden Bedingungen auf ein 12prozentiges Polyacrylamid-SDS-Gel gegeben und der Gelelektrophorese unterzogen. Anschließend werden die nach ihrem Molekulargewicht getrennten G-CSF-Monomere, -Dimere oder -Aggregate durch Elektroblotting auf Nitrocellulose transferiert. Durch Inkubation mit einem spezifischen polyklonalen biotinylierten Anti-G-CSF-Antikörper (PAK < GCSF >IgG) werden die Proteinbanden identifiziert und mittels der Phosphatase-Technik unter Benutzung von Streptavidin-alkalischem Phosphatasekonjugat (SA-AP-Konjugat), 5-Brom-4-chlor-3-indolylphosphat (BCIP) und Nitro Blue Tetrazolin (NBT) nachgewiesen.
   Die Bestimmung der prozentualen Monomeren-, Dimeren- bzw. Aggregatanteile erfolgt durch laserdensitometrische Auswertung mit Hilfe einer rhG-CSF-Standardreihe.
2.4 NFS-60 Bioassay (biologische Wirksamkeit)
   Die in vitro Aktivitätsbestimmung von G-CSF basiert auf der Messung von Zellzahlen in einer durch G-CSF stimulierten Zellkultur von NFS-60 Zellen.
   Unter geeigneten Bedingungen kann die Dehydrogenase-Aktivität der Zellen mit der Konzentration an G-CSF im Medium korreliert werden. Es werden geeignete Verdünnungen der G-CSF Pufferlösung hergestellt, um einen einfach meßbaren Anstieg in der Dehydrogenase-Aktivität zu erhalten.
   Die Messung der Aktivität erfolgt dann photometrisch bei 570 und 690 nm, gemessen wird die Reduktion des Tetrazolinium-Salzes MTT (gelb) zu Formazan (blau).
   Die in vitro Aktivität von G-CSF wird berechnet, indem die Daten der Probe gegen Standard nach der Methode der parallelen Linie verglichen werden. Die Auswertung erfolgt gemäß den Anforderungen der Ph. Eur. (VIII, 13).
2.5 Photometrie OD 280 (Proteingehalt)
   Das G-CSF-UV-Spektrum hat ein Absorptionsmaximum bei 280 nm, das auf Seitenkettenchromophore wie Tryptophan-, Tyrosin- und Phenylalaninreste zurückzuführen ist. Die Messung erfolgt im Vergleich zu Placebo-Lösungen mittels:
   - UV-Spectrophotometer
      (z. B. Uvikon 810 P oder 941, Kontron Instruments)
   - Semi-micro Quartz Küvetten, 500 µl, Schichtdicke: 1 cm (z. B. Hellma, Suprasil, Cat. No. 104.002B-QS)
2.6. Streulichtmessung, Trübungsmessung
   Die Messung erfolgt direkt an der unverdünnten Produktlösung in Glasküvetten (Durchmesser 2 cm). Das von der Flüssigkeit diffus abgelenkte Streulicht wird unter einem Winkel von 90 C gemessen. Gemessen wird im Vergleich zu Formazin-Standard-Suspensionen nach DIN 38404C2, die Angabe der Werte erfolgt in TE/F. Die Messung erfolgt an einem geeigneten Trübungsphotometer, z. B. LTP 5 (Fa. Dr. Lange, Düsseldorf).

### Beispiel 3:

### Untersuchungen zur mechanischen Belastbarkeit (Turbidität)

G-CSF-Lösungen bei einer Konzentration von 0,5 mg/1ml wurden durch Dialyse mit den nachfolgend genannten Puffern hergestellt. Zur Ermittlung der nach mechanischem Streß auftretenden Trübungen wurden Proben von jeweils 1 ml in Injektionsflaschen aus Glas der hydrolytischen Klasse 1 abgefüllt und mit Stopfen verschlossen. Die Proben wurden 10Sek. bei maximaler Intensität auf Laborschüttlern (z.B. Firma Heidolph) behandelt. Direkt nach dem mechanischen Streßvorgang folgte die Streulichtmessung mit einem Hitachi F4000-Fluoreszenzspektrophotometer bei einer EX- und EM-Wellenlänge von jeweils 360 nm. Angegeben werden nachfolgend die Streulichtintensitäten bei 360nm in Abhängigkeit vom pH-Wert und der eingesetzten Puffer. Die angegebenen Molaritäten des Acetat- und Citratpuffers beziehen sich auf die vorgelegten Molaritäten Essigsäure und Zitronensäure. Die entsprechenden pH-Werte wurden mit Natronlaugen eingestellt. Phosphat-/Citratpuffer wurde durch Vorlegen der entsprechenden Menge Zitronensäure und pH-Einstellung mit Dinatriumhydrogenphosphat hergestellt. Phosphatpuffer wurden durch Vorlage der gegebenen Molaritäten an Natriumdihydrogenphosphat und Einstellen der pH-Werte mit Phosphorsäure bzw. Dinatriumhydrogenphosphat hergestellt.

### Beispiel 4:

Wie in Beispiel 1 angegeben wird eine flüssige Arzneiform von G-CSF hergestellt und der pH-Wert der Lösung auf 4,5 eingestellt. Die Zubereitungen enthalten im einzelnen die in der Tabelle 1 angegebenen Bestandteile:

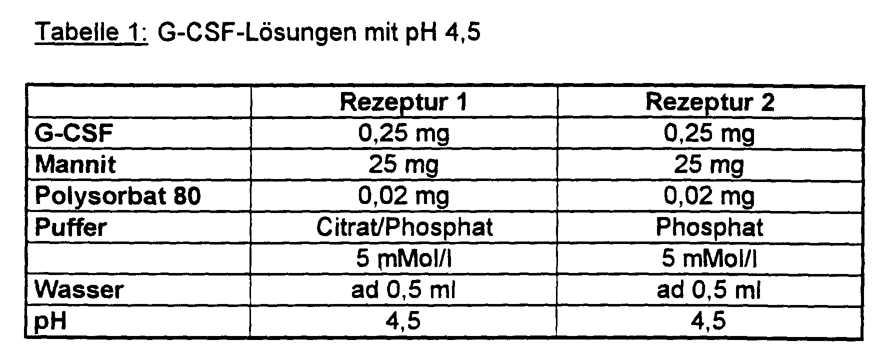

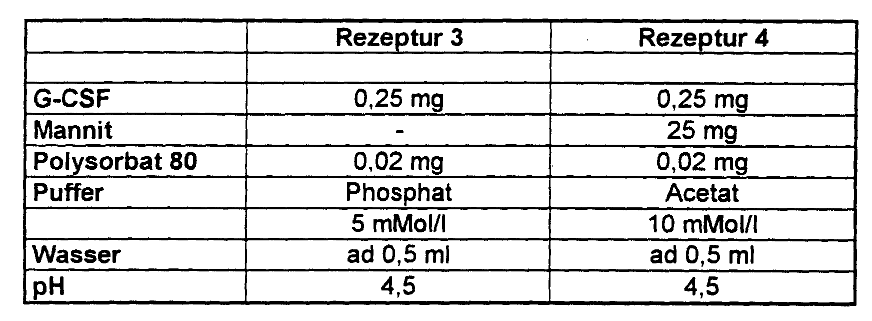

### a) Ergebnisse bezüglich Proteinreinheit bei ruhiger Lagerung

Die Analysen der flüssigen Rezepturen 1 - 4 durch RP HPLC, SEC HPLC und Western Blot zeigen, daß die Reinheit des unveränderten Proteins > 99 % beträgt und keine Dimere oder Aggregate von G-CSF nachweisbar sind. Diese Ergebnisse wurden erhalten nach Lagerung der Rezepturen 1 - 4 über sechs Monate bei 4 - 8 °C, nach Lagerung über vier Wochen bei + 30 °C und nach Lagerung über vier Wochen bei + 40 °C. Die Ergebnisse zeigen, daß Lösungen von G-CSF bei einem pH von 4,5 und unterschiedlichen Lagerbedingungen hinsichtlich ruhiger Lagerbedingungen als weitgehend stabil gekennzeichnet werden können.

### b) Ergebnisse bezüglich Proteinreinheit nach mechanischer Belastung

Die Auswahl eines Puffers und des geeigneten pH-Wertes der Lösung für die chemisch-physikalische Stabilität bei ruhiger Lagerung ist jedoch unterschiedlich im Hinblick auf die Stabilität nach mechanischer Belastung. Bei pH 4,5 ist diese Stabilität in Acetat- und Phosphatpuffem gegeben, nicht aber in einer Puffermischung von Citrat/Phosphat. Die erhaltenen Ergebnisse sind im einzelnen der folgenden Tabelle zu entnehmen:

**Tabelle 2:**

| | **Trübung nach 6 Monaten bei 4 - 8 °C** | **Trübung nach mechanischem Streß** | | |
|---|---|---|---|---|
| | | ***direkt nach*** ***Herstellung*** | ***bei*** ***4 - 8 °C*** | ***bei*** ***40 °C*** |
| | | | | |
| **Rez. 1** | keine | stark | stark | stark |
| **Rez. 2** | keine | keine | keine | keine |
| **Rez. 3** | keine | keine | keine | keine |
| **Rez. 4** | keine | keine | keine | keine |

### Beispiel 5:

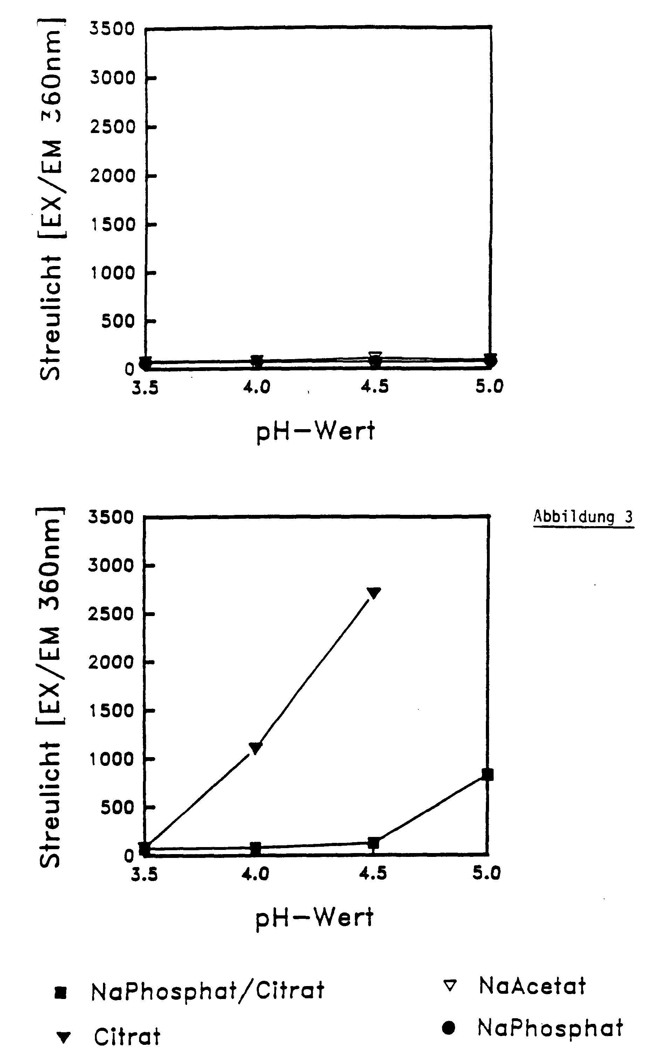

Wie in Beispiel 3 beschrieben wurden Lösungen von G-CSF mit verschiedenen Puffersystemen und jeweils 0,005 % (in Abb.2 und 3 0,05 %) Polysorbat 80 hergestellt. Für jedes untersuchte Puffersystem wurden verschiedene Lösungen mit unterschiedlichen pH-Werten zwischen 2 - 7,5 in Abständen von 0,5 pH-Einheiten hergestellt (somit pro Puffersystem 13 Lösungen). Nach den oben beschriebe- nen Methoden (Beispiel 3) wurde die mechanische Belastbarkeit der Lösungen mittels Streulichtmessung untersucht. Folgende Puffersysteme wurden untersucht (Gehalt an G-CSF und übrigen Hilfsstoffe wie in Beispiel 3):

| | |
|---|---|
| 5.1 | Acetat 10 mMol/l |
| 5.2 | Acetat 20 mMol/l |
| 5.3 | Acetat 40 mMol/l |
| 5.4 | Phosphat 20 mMol/l |
| 5.5 | Phosphat/Citrat insgesamt 20 mMol/l, mit 10 mMol/l Phosphat |
| 5.6 | Lactat 10 mMol/l |
| 5.7 | Maleat 10 mMol/l |
| 5.8 | Citrat 20 mMol/l |

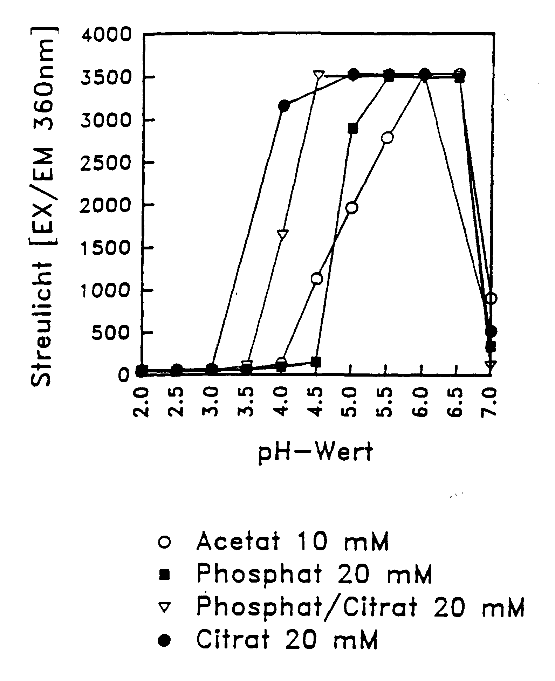

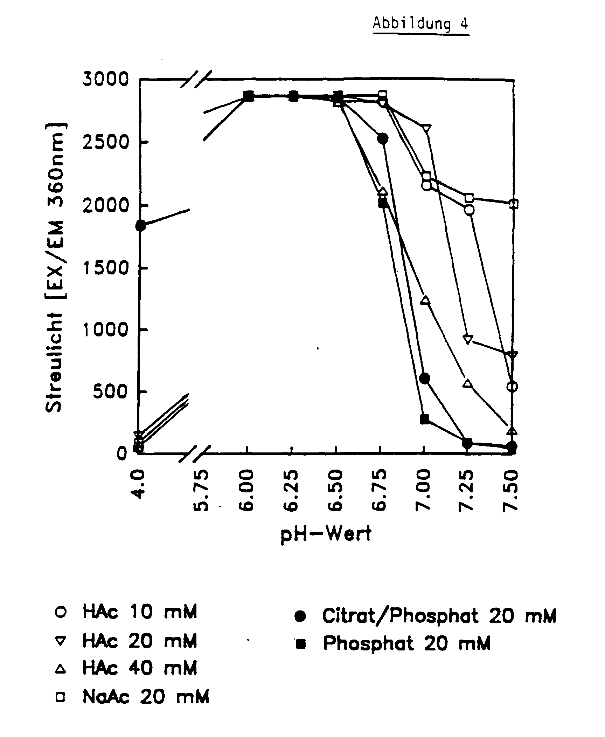

In Abhängigkeit des pH-Wertes der Lösung erfolgte die Messung des Streulichts nach erfolgter mechanischer Belastung. Die Ergebnisse sind den Abb. 1 - 4 zu entnehmen. Für jedes Puffersystem ist dort ein individueller Verlauf der pH-Trübungskurve angegeben. Citrat ist insbesondere im pH-Bereich von 4 - 6,5 ungeeignet; Citrat/Phosphatpuffer im pH-Bereich von 4,5 - 6,5 ungeeignet; Acetat im pH-Bereich von etwa 5 - 7 ungeeignet und Phosphat im pH-Bereich von etwa 5 -6,75 ungeeignet. Lactat verhält sich wie Acetat, Maleat wie Citrat/Phosphat,

### Beispiel 6:

### Untersuchungen zur mechanischen Stabilität von G-CSF-Lösungen einer Konzentration von 0,35 mg/ml mit 0,5 mg/ml Tween 80 in Abhängigkeit von Pufferkonzentration und pH-Wert

G-CSF-Lösungen einer Konzentration von ca. 5 mg/ml wurden mit nachfolgend beschriebenen Pufferlösungen auf einen Wirkstoffgehalt von 0,35 mg/ml verdünnt. Alle Pufferlösungen enthielten 0,05 % Polysorbat 80. Die Pufferlösungen wurden hergestellt, indem die jeweilige basische Salzkomponente in der angegebenen molaren Konzentration vorgelegt und mit der korrespondierenden Säure der pH-Wert auf den angegebenen Wert eingestellt wurde. Die so erhaltenen G-CSF-Pufferlösungen wurden in einer Menge von 10 ml in Injektionsflaschen aus Glas der hydrolytischen Klasse 1 eingefüllt, mit einem geeigneten Stopfen verschlossen und 10 Sekunden bei maximaler Intensität auf einem Laborschüttler (z. B. Fa. Heidolph) geschüttelt. Nach einer Standzeit von ca. 10 Minuten wurde mit einem Streulichtphotometer (Meßwinkel 90 °C) der Firma Dr. Lange, Streulichtphotometer LTP 5 ein gegen Formazinstandard (Deutsches Arzneibuch) kalibrierter Trübungswert ermittelt (TE/F).

**Tabelle 3a:**

| Trübung der Lösung ungeschüttelt | | | | |
|---|---|---|---|---|
| | | **Acetat** **Puffer** | **Phosphat** **Puffer** | **Citrat** **Puffer** |
| | | **TE/F** | **TE/F** | **TE/F** |
| | **pH 7,5** | 0,56 | 0,58 | 0,51 |
| | **pH 7** | 0,47 | 0,51 | 0,43 |
| **80 mmol** | **pH 5** | 0,55 | 0,47 | 0,48 |
| | **pH 4,5** | 0,36 | 0,47 | 0,46 |
| | **pH 4** | 0,39 | 0,46 | 0,41 |
| | **pH 3,5** | 0,3 | 0,54 | 0,41 |
| | **pH 7,5** | 0,57 | 0,4 | 0,45 |
| | **pH 7** | 0,51 | 0,37 | 0,39 |
| **40 mmol** | **pH 5** | 0,62 | 0,4 | 0,48 |
| | **pH 4,5** | 0,42 | 0,58 | 0,52 |
| | **pH 4** | 0,44 | 0,41 | 0,55 |
| | **pH 3,5** | 0,42 | 0,43 | 0,37 |
| | **pH 7,5** | 0,65 | 0,39 | 0,4 |
| | **pH 7** | 0,8 | 0,46 | 0,4 |
| **20 mmol** | **pH 5** | 0,62 | 0,49 | 0,39 |
| | **pH 4,5** | 0,47 | 0,42 | 0,39 |
| | **pH 4** | 0,44 | 0,43 | 0,47 |
| | **pH 3,5** | 0,49 | 0,37 | 0,38 |
| | **pH 7,5** | 0,54 | 0,43 | 0,55 |
| | **pH 7** | 0,68 | 0,38 | 0,49 |
| **10 mmol** | **pH 5** | 0,42 | 0,41 | 0,55 |
| | **pH 4,5** | 0,37 | 0,45 | 0,43 |
| | **pH 4** | 0,35 | 0,36 | 0,44 |
| | **pH 3,5** | 0,4 | 0,43 | 0,42 |
| | **pH 7,5** | 0,83 | 0,57 | 0,35 |
| | **pH 7** | 0,63 | 0,37 | 0,37 |
| **5 mmol** | **pH 5** | 0,65 | 0,41 | 0,42 |
| | **pH 4,5** | 0,42 | 0,4 | 0,31 |
| | **pH 4** | 0,4 | 0,4 | 0,64 |
| | **pH 3,5** | 0,38 | 0,48 | 0,44 |

**Tabelle 3b:**

| Trübung der Lösung geschüttelt | | | | |
|---|---|---|---|---|
| | | **Acetat** **Puffer** | **Phosphat** **Puffer** | **Citrat** **Puffer** |
| | | **TE/F** | **TE/F** | **TE/F** |
| | **pH 7,5** | 1,49 | 0,74 | 0,8 |
| | **pH 7** | 1,94 | 0,77 | 0,79 |
| **80 mmol** | **pH 5** | 0,73 | 0,75 | 2,8 |
| | **pH 4,5** | 0,42 | 0,68 | 2,1 |
| | **pH 4** | 0,45 | 0,57 | 0,81 |
| | **pH 3,5** | 0,32 | 0,66 | 0,57 |
| | **pH 7,5** | 1,52 | 0,7 | 0,74 |
| | **pH 7** | 1,24 | 0,58 | 0,57 |
| **40 mmol** | **pH 5** | 0,8 | 0,6 | 1,69 |
| | **pH 4,5** | 0,5 | 0,64 | 0,89 |
| | **pH 4** | 0,63 | 0,62 | 0,89 |
| | **pH 3,5** | 0,55 | 0,64 | 0,47 |
| | **pH 7,5** | 2,2 | 0,45 | 0,57 |
| | **pH 7** | 2,9 | 0,6 | 0,57 |
| **20 mmol** | **pH 5** | 0,7 | 0,77 | 0,95 |
| | **pH 4,5** | 0,55 | 0,48 | 0,6 |
| | **pH 4** | 0,56 | 0,5 | 0,74 |
| | **pH 3,5** | 0,43 | 0,4 | 0,55 |
| | **pH 7,5** | 1,22 | 0,59 | 0,75 |
| | **pH 7** | 1,4 | 0,46 | 0,65 |
| **10 mmol** | **pH 5** | 0,47 | 0,51 | 1,35 |
| | **pH 4,5** | 0,43 | 0,45 | 0,59 |
| | **pH 4** | 0,47 | 0,38 | 0,58 |
| | **pH 3,5** | 0,44 | 0,51 | 0,46 |
| | **pH 7,5** | 2 | 0,76 | 0,48 |
| | **pH 7** | 1,4 | 0,6 | 0,69 |
| **5 mmol** | **pH 5** | 0,47 | 0,51 | 0,69 |
| | **pH 4,5** | 0,43 | 0,44 | 0,44 |
| | **pH 4** | 0,47 | 0,44 | 0,81 |
| | **pH 3,5** | 0,44 | 0,5 | 0,44 |

Aus den Daten geht hervor, daß Phosphatpuffer im untersuchten pH-Bereich über die gesamte Molarität von 5 - 80 mMol/l niedrige Trübungswerte zeigen, die unter mechanischer Belastung geringfügig ansteigen.

Acetatpuffer zeigen im pH-Bereich von 3,5 - 4,5 auch unter mechanischem Streß sehr geringe Trübungswerte. Citratpuffer sind geeignet, im pH-Bereich von 7 - 7,5 in Konzentrationen von 5 - 40 mMol/l zu stabilisieren.

### Beispiel 7:

Es werden flüssige Rezepturen im pH-Bereich von 7,0 - 7,5 beschrieben, denen zum Teil neben isotonisierenden Zusätzen auch weitere Hilfsstoffe aus der Gruppe der Antioxidatien und chaotrope Hilfsstoffe zugesetzt werden.

Zur Herstellung der nachfolgend genannten Rezepturen wurden die entsprechenden Hilfsstoffe in Wasser für Injektionszwecke gelöst, G-CSF wurde zugefügt und gegebenenfalls der pH-Wert durch geringe Mengen der entsprechenden Pufferkomponenten genau eingestellt. Die Lösungen wurden durch einen sterilisierten Membranfilter mit der Porenweite von 0,2 µm sterilfiltriert und unter aseptischen Bedingungen in sterile Injektionsflaschen aus Glas der hydrolytischen Klasse I gefüllt und mit sterilen, teflonisierten Gummistopfen verschlossen. Die Abfüllung fand unter Stickstoffbegasung statt. Die verbördelten Injektionsflaschen wurden unter Lichtausschluß bei definierten Lagertemperaturen bis zur Untersuchung gelagert. Untersucht wurde mit den in den Beispielen 2 und 3 beschriebenen Methoden.

**Tabelle 4a:**

| Flüssige G-CSF Zubereitung; pH 7 | | | |
|---|---|---|---|
| | **Rezeptur 5** | **Rezeptur 6** | **Rezeptur 7** |
| **G-CSF** | 0,35 mg | 0,35 mg | 0,35 mg |
| **Polysorbat** | 0,1 mg | 0,1 mg | 0,1 mg |
| **Mannit** | 40 mg | 40 mg | 40 mg |
| **Puffer** | Na₂HPO₄12H₂O 1,5 mg | Na₂HPO₄12H₂O 1,5 mg | Na₂HPO₄12H₂O 1,5 mg |
| **Glutathion** | - | 0,5 mg | - |
| **Harnstoff** | - | - | 5 mg |
| **Wasser für Injektionszwecke** | ad 1 ml | ad 1 ml | ad ml |

**Tabelle 4b:**

| Analysendaten zu Flüssigrezepturen zu G-CSF bei pH 7 | | | |
|---|---|---|---|
| | **Lagerung 4 Wochen bei 4 - 8 °C** | | |
| | **I** | **II** | **III** |
| | | | |
| **Rez. 5** | >99 % | > 99 % | 2,7 % |
| **Rez. 6** | 99 % | > 99 % | 4,8 % |
| **Rez. 7** | > 99 % | > 99 % | 0,6 % |

I Reinheit unverändertes Protein in RP HPLC
II Reinheit unverändertes Protein in SEC HPLC
III Dimeren/Aggregate in Western Blot

Die Analysendaten aus Tabelle 4b zeigen, daß stabile Lösungen bei pH 7 und der angegebenen Phosphatpufferkonzentration erhalten werden und die Reinheit des Proteins nach vier Wochen Lagerung weitgehend unverändert (> 99 %) beibehalten wird.

### Beispiel 8:

Es werden Lösungen von G-CSF hergestellt, wobei eine Lösung Phosphatpuffer enthält und einen erfindungsgemäßen pH-Wert von 4,5 besitzt (Rezeptur 8). Zu Vergleichszwecken wird eine zweite Lösung hergestellt, die einen pH-Wert von 6,5 aufweist (Rezeptur 9).

Zur Herstellung der Arzneistofflösungen wurden die Hilfsstoffe in Wasser für Injektionszwecke gelöst, dann wurde GCSF zugefügt. Die Lösungen wurden durch einen sterilen 0,2 µm Membranfilter sterilfiltriert, in Vials der hydrolytischen Klasse I abgefüllt und diese mit einem Teflonstopfen verschlossen. Danach wurde 10 Min. mit einem Laborschüttler (Fa. Heidolph) behandelt. Untersucht wurde die Lösung mit den in den Beispielen 2 und 3 beschriebenen Methoden.

**Tabelle 5a:**

| Zusammensetzung der Rezepturen | | |
|---|---|---|
| | **Rezeptur 8** | **Rezeptur 9** |
| | | |
| **G-CSF** | 0,350mg | 0,250 mg |
| **Tween 80** | 0,1 mg | - |
| **Tween 20** | - | 0,1 mg |
| **Humanserum- albumin** | - | 1 mg |
| **Mannit** | 50 mg | 50 mg |
| **Phosphatpuffer** | 5 mMol/l | 5 mMol/l |
| **pH** | 4,5 | 6,5 |
| **Wasser für Injektionszwecke** | ad 1.0 ml | ad 1,0 ml |

**Tabelle 5b:**

| Analytische Ergebnisse (Western Blot) | | |
|---|---|---|
| | **Rezeptur 8** | **Rezeptur 9** |
| | | |
| **ohne mechanische Belastung** | 0,8 % Dimere keine Aggregate | 1,1 % Dimere keine Aggregate |
| | | |
| **nach mechanischer Belastung** | 1,1 % Dimere keine Aggregate | 2,7 % Dimere 6,9 % Aggregate |

Aus den in der Tabelle angegebenen Ergebnissen ist ersichtlich, daß auch der Zusatz von Human-Serum-Albumin die durch mechanische Belastung hervorgerufene Aggregation und Dimerisierung des Proteins bei pH 6,5 nicht verhindern kann. Bei einem pH-Wert von 4,5 hingegen wird eine gegenüber mechanischer Belastung stabile Lösung erhalten, auch wenn kein Humanserumalbumin als Stabillsierungsmittel vorhanden ist.

### Beispiel 9:

### Langzeitstabilität

Eine G-CSF-lnjektionslösung wurde hergestellt, indem man 0,35 mg/ml G-CSF in einer Lösung von 50 mg/ml Manitol, 0,1 mg/ml Polysorbat 80 und 0,5 mg Phosphorsäure, eingestellt auf pH 4,5 mit Natriumhydrogenphosphat unter Rühren löst. Herstellung und Abfüllung der Lösung erfolgte unter Stickstoffbegasung. Die klare Lösung wurde durch Filtration mittels sterilisierter Membranfilter mit der Porengröße 0,2 µm sterilfiltriert, anschließend in Injektionsflaschen der hydrolytischen Klasse 1 abgefüllt und mit geeigneten Gummistopfen verschlossen. Die G-CSF-haltigen Arzneizubereitungen wurden bei Temperaturen von + 4 bis + 8 sowie + 20 bis + 25 C über 9 Monate gelagert.

**Tabelle 6:**

| Langzeitstabilität bei 4 - 8 C | | | |
|---|---|---|---|
| | **3 Monate** | **6 Monate** | **9 Monate** |
| | | | |
| **biol. Aktivität** | | | |
| **80 %-125 %** | entspr. | entspr. | entspr. |
| **Western Blot** | | | |
| **% Aggr.** | n.n. | n.n. | n.n. |
| **% Dimere** | < 1 % | < 1 % | < 1 % |
| **SDS Page** | | | |
| **% Nebenpeaks** | < 1 % | < 1 % | < 1 % |
| **RP-HPLC** | | | |
| **Produktpeak** | > 99 % | > 99 % | > 99 % |
| **SEC HPLC** | | | |
| **Prod. peak** | > 98 % | > 98 % | > 98 % |
| **Nebenpeaks** | < 1 % | < 1 % | < 1 % |
| **Trübung TE/F** | 0,7 | 0,7 | 0,7 |
| **OD 280** | 0,359 | 0,362 | 0,357 |

### Beispiel 10:

G-CSF-Lösungen einer Konzentration von ca. 5 mg/ml wurden mit nachfolgend beschriebenen Pufferlösungen auf einen Wirkstoffgehalt von 0,35 mg/ml verdünnt. Alle Pufferlösungen enthielten 0,05 % Polysorbat 80. Die Pufferlösungen wurden hergestellt, indem Arginin in der angegebenen molaren Menge zwischen 5 - 80 mmol/l vorgelegt und mit Phosphorsäure oder Salzsäure oder Citronensäure der pH-Wert eingestellt wurde. Die so erhaltenen G-CSF-Pufferlösungen wurden in einer Menge von 10 ml in Injektionsflaschen aus Glas der hydrolytischen Klasse 1 eingefüllt, mit einem geeigneten Stopfen verschlossen und 10 Sekunden bei maximaler Intensität auf einem Laborschüttler (z. B. Fa. Heidolph) geschüttelt. Nach einer Standzeit von ca. 10 Minuten wurde mit einem Streulichtphotometer (Meßwinkel 90 °C) der Firma Dr. Lange, Streulichtphotometer LTP 5 ein gegen Formazinstandard (Deutsches Arzneibuch) kalibrierter Trübungswert ermittelt (TE/F).

In Tabelle 7 sind die Trübungsmittelwerte der ungeschüttelten und geschüttelten Lösungen dargestellt. Die verschiedenen molaren Konzentrationen (5, 10, 20, 40, 80 mMol) an Argininpuffersystemen führen zu jeweils gleichen Ergebnissen.

Es ist festzustellen, daß Argininpuffer im pH-Bereich von 7,0 - 7,5 ganz besonders gut stabilisierend gegen mechanischen Stress wirkt. Argininpuffer können besonders vorteilhaft in Lösungen eingesetzt werden, die durch Auflösen von Lyophilisaten hergestellt werden.

**Tabelle 7:**

| Trübungswerte mit Argininpuffer | | | |
|---|---|---|---|
| **Puffer** | **Arginin/ Phosphorsäure** | **Arginin/ Salzsäure** | **Arginin/ Citronensäure** |
| **pH 7,5** | 0,40 | 0,42 | 0,51 |
| **pH 7,0** | 0,43 | 0,54 | 0,76 |
| **ungeschüttelt** | | | |
| | | | |
| **pH 7,5** | 0,49 | 0,62 | 0,53 |
| **pH 7,0** | 0,58 | 0,69 | 0,84 |
| **geschüttelt** | | | |

Mittelwerte Molarität 5 - 80 mMol/l

## Patentansprüche

1. Stabile, wäßrige pharmazeutische Zubereitung enthaltend G-CSF in einer therapeutisch wirksamen Menge, enthaltend eine Tensidmenge, die kleiner der eingesetzten Menge an G-CSF ist, und enthaltend mindestens eine Puffersubstanz ausgewählt aus der Gruppe Essigsäure, Milchsäure, Citronensäure, Maleinsäure, Phosphorsäure und deren Salze oder Arginin und seiner Salze, wobei die Endkonzentration der Puffersubstanz in der fertig applizierbaren Lösung von 2 bis 100 mMol/l beträgt.

2. Zubereitung gemäß Anspruch 1, dadurch gekennzeichnet, daß die Konzentration von Phosphat 5-80 mMol/l beträgt und der pH-Wert der fertig applizierbaren Lösung auf Werte zwischen etwa 3,5 - 5 oder 7 - 8 eingestellt ist.

3. Zubereitung gemäß Anspruch 1, dadurch gekennzeichnet, daß die Konzentration von Citrat oder die Konzentration von Maleat 5-40 mMol/l beträgt und der pH-Wert der Lösung auf Werte zwischen 2,5 - 3,5 oder 7 - 6 eingestellt ist.

4. Zubereitung gemäß Anspruch 1, dadurch gekennzeichnet, daß die Gesamtkonzentration von Phosphat und Citrat als Puffersubstanzen 5 - 40 mMol/l beträgt und der pH-Wert der Lösung auf Werte zwischen 2,5 - 3,5 oder 7 - 8 eingestellt ist.

5. Zubereitung gemäß Anspruch 1, dadurch gekennzeichnet, daß die Konzentration von Acetat oder die Konzentration von Lactat 5 - 80 mMol/l beträgt und der pH-Wert der Lösung auf Werte zwischen etwa 3,5 - 5 eingestellt ist.

6. Zubereitung gemäß Anspruch 1, dadurch gekennzeichnet, daß die Konzentration von Argininphosphat-, Argininchlorid- oder Arginincitratpuffer 5 - 80 mMol/l beträgt und der pH-Wert der Lösung auf Werte zwischen etwa 7 - 8, vorzugsweise 7 - 7,5 eingestellt ist.

7. Zubereitung gemäß Anspruch 1, dadurch gekennzeichnet, daß die Lösung eine Tensidmenge enthält, die maximal 0,5 mg/ml, vorzugsweise 0,01 - 0,1 mg/ml, beträgt.

8. Zubereitung gemäß einem der Ansprüche 1 - 7, dadurch gekennzeichnet, daß die Lösung zusätzlich übliche pharmazeutische Hilfsstoffe oder isotonisierende Mittel enthält.

9. Zubereitung gemäß einem der Ansprüche 1 - 8, dadurch gekennzeichnet, daß die Lösung frei von polymeren oder proteinartigen Hilfsstoffen ist.

10. Verfahren zur Herstellung einer stabilen wäßrigen pharmazeutischen Zubereitung enthaltend G-CSF, dadurch gekennzeichnet, daß man eine pharmazeutisch wirksame Menge von G-CSF mit einer Tensidmenge, die kleiner der eingesetzten Menge an G-CSF ist, und mit mindestens einer Puffersubstanz ausgewählt aua der Gruppe Essigsäure, Milchsäure, Citronensäure, Maleinsäure, Phosphorsäure und deren Salze oder Arginin und seiner Salze versetzt, und die Menge der Puffersubstanz derart wählt, daß die Endkonzentration der Puffersubstanz in der fertig appleierbaren Lösung von 2 bis zu 100 mMol/l beträgt.

11. Verfahren zur Stabilisierung einer wäßrigen pharmazeutischen Zubereitung enthaltend G-CSF, dadurch gekennzeichnet, daß man neben einer Tensidmenge, die kleiner der eingesetzten Menge an G-CSF ist, mindestens eine Puffersubstanz ausgewählt aus der Gruppe Essigsäure, Milchsäure, Citronensäure, Maleinsäure, Phosphorsäure und deren Salze oder Arginin und seiner Salze in einer Endkonzentration von 2 bis zu 100 mMol/l in der fertig applizierbaren Lösung als Stabilisierungsmittel zur Vermeidung von Trübungen einsetzt.

12. Verwendung von Essigsäure, Mllchsäure, Citronensäure, Maleinsäure, Phosphorsäure und deren Salzen oder Arginin und seiner Salze als Puffersubstanzen zur Vermeidung von Trübungen im Fall von wäßrigen pharmazeutischen G-CSF-haltigen Zubereitungen, wobei die Endkonzentration der Puffersubetanzen in der fertig applizierbaren Lösung von 2 - 100 mMol/l beträgt und die Puffersubstanzen zusammen mit einer Tensidmenge eingesetzt werden, die kleiner der eingesetzten Menge an G-CSF ist.

13. Lyophilisat oder Pulver, hergestellt aus einer Zubereitung gemäß einem der Ansprüche 1 - 9.

14. Zubereitung gemäß einem der Ansprüche 1-9, hergestellt durch Auflösen von Lyophillsaten oder Pulvern gemäß Anspruch 13 in Wasser oder wäßrigen Lösungen.

## Claims

1. Stable aqueous pharmaceutical preparation containing G-CSF in a therapeutically effective amount containing an amount of surfactant which is smaller than the amount of G-CSF used and containing at least one buffer substance selected from the group comprising acetic acid, lactic acid, citric acid, maleic acid, phosphoric acid and salts thereof or arginine and its salts, wherein the final concentration of the buffer substance in the ready-to-administer solution is from 2 to 100 mmol/l.

2. Preparation as claimed in claim 1, wherein the concentration of phosphate is 5 - 80 mmol/l and the pH value of the ready-to-administer solution is adjusted to values between 3.5 to 5 or 7 to 8.

3. Preparation as claimed in claim 1, wherein the concentration of citrate or the concentration of maleate is 5 - 40 mmol/l and the pH value of the solution is adjusted to values between 2.5 to 3.5 or 7 to 8.

4. Preparation as claimed in claim 1, wherein the total concentration of phosphate and citrate as buffer substances is 5 - 40 mmol/l and the pH value of the solution is adjusted to values between 2.5 to 3.5 or 7 to 8.

5. Preparation as claimed in claim 1, wherein the concentration of acetate or the concentration of lactate is 5 - 80 mmol/l and the pH value of the solution is adjusted to values between about 3.5 and 5.

6. Preparation as claimed in claim 1, wherein the concentration of arginine phosphate, arginine chloride or arginine citrate buffer is 5 - 80 mmol/l and the pH value of the solution is preferably adjusted to values between 7 to 8, and more preferably between 7 to 7.5.

7. Preparation as claimed in claims 1, wherein the solution contains an amount of surfactant which is at most 0.5 mg/ml, preferably 0.01 - 0.1 mg/ml.

8. Preparation as claimed in one of the claims 1 -7, wherein the solution in addition contains common auxiliary substances or isotonizing agents.

9. Preparation as claimed in one of the claims 1 - 8, wherein the solution is free of polymers or protein-like auxiliary substances.

10. Process for the preparation of a stable aqueous pharmaceutical preparation containing G-CSF, wherein a pharmaceutically effective amount of G-CSF is admixed with an amount of surfactant which is smaller than the amount of G-CSF used, and with at least one buffer substance selected from the group comprising acetic acid, lactic acid, citric acid, maleic acid, phosphoric acid and salts thereof or arginine and its salts and the amount of buffer substance is selected in such a way that the final concentration of the buffer substance in the ready-to-administer solution is 2 to 100 mmol/l.

11. Process for stabilizing an aqueous pharmaceutical preparation containing G-CSF, wherein apart from an amount of surfactant which is smaller than the amount of G-CSF used, at least one buffer substance selected from the group comprising acetic acid, lactic acid, citric acid, maleic acid, phosphoric acid and salts thereof or arginine and its salts is used in a final concentration of 2 to 100 mmol/l in the ready-to-administer solution as a stabilizing agent to prevent turbidities.

12. Use of acetic acid, lactic acid, citric acid, maleic acid, phosphoric acid and salts thereof or arginine and its salts as buffer substances for the prevention of turbidities in the case of aqueous pharmaceutical preparations containing G-CSF wherein the final concentration of the buffer substances in the ready-to-administer solution is 2 - 100 mmol/l and the buffer substances are used together with an amount of surfactant which is smaller than the amount of G-CSF used.

13. Lyophilisate or powder produced from a preparation as claimed in one of the claims 1 - 9.

14. Preparation as claimed in one of the claims 1 - 9, produced by dissolving lyophilisates or powders as claimed in claim 13 in water or aqueous solutions.

## Revendications

1. Préparation pharmaceutique aqueuse stable contenant du G-CSF en une quantité à effet thérapeutique, contenant une quantité d'un tensioactif inférieure à la quantité utilisée de G-CSF, et contenant au moins une substance tampon choisie dans l'ensemble comprenant l'acide acétique, l'acide lactique, l'acide citrique, l'acide maléique, l'acide phosphorique et leurs sels, ou l'arginine et ses sels, la concentration finale de la substance tampon dans la solution pouvant être appliquée à l'état fini étant de 2 à 100 mmol/l.

2. Préparation selon la revendication 1, caractérisée en ce que la concentration du phosphate est de 5 à 80 mmol/l et le pH de la solution pouvant être appliquée à l'état fini est ajusté à des valeurs comprises entre environ 3,5 et 5, ou 7 et 8.

3. Préparation selon la revendication 1, caractérisée en ce que la concentration du citrate ou la concentration du maléate est de 5 à 40 mmol/l, et le pH de la solution est ajusté à des valeurs comprises entre 2,5 et 3,5 ou entre 7 et 8.

4. Préparation selon la revendication 1, caractérisée en ce que la concentration totale du phosphate et du citrate en tant que substances tampon est de 5 à 40 mmol/l, et le pH de la solution est ajusté à des valeurs comprises entre 2,5 et 3,5 ou entre 7 et 8.

5. Préparation selon la revendication 1, caractérisée en ce que la concentration de l'acétate ou la concentration du lactate est de 5 à 80 mmol/l, et le pH de la solution est ajusté à des valeurs comprises entre environ 3,5 et 5.

6. Préparation selon la revendication 1, caractérisée en ce que la concentration du tampon phosphate d'arginine, chlorure d'arginine ou citrate d'arginine est de 5 à 80 mmol/l, et le pH de la solution est ajusté à des valeurs comprises entre environ 7 et 8, de préférence entre 7 et 7,5.

7. Préparation selon la revendication 1, caractérisée en ce que la solution contient une quantité d'un tensio-actif qui est au maximum de 0,5 mg/ml et de préférence de 0,01 à 0,1 mg/ml.

8. Préparation selon l'une des revendications 1 à 7, caractérisée en ce que la solution contient en outre des adjuvants pharmaceutiques ou des agents d'isotonicité.

9. Préparation selon l'une des revendications 1 à 8, caractérisée en ce que la solution est exempte d'adjuvants polymères ou de type protéine.

10. Procédé pour fabriquer une préparation pharmaceutique aqueuse stable contenant du G-CSF, caractérisé en ce qu'on ajoute à une quantité à effet pharmaceutique de G-CSF une quantité d'un tensioactif qui est inférieure à la quantité utilisée de G-CSF, et au moins une substance tampon choisie dans l'ensemble comprenant l'acide acétique, l'acide lactique, l'acide citrique, l'acide maléique, l'acide phosphorique et leurs sels, ou l'arginine et ses sels, et on choisit la quantité de la substance tampon de façon que la concentration finale de la solution tampon dans la solution pouvant être appliquée à l'état fini soit de 2 à 100 mmol/l.

11. Procédé pour stabiliser une préparation pharmaceutique aqueuse contenant du G-CSF, caractérisé en ce qu'on utilise en tant que stabilisant, pour éviter des phénomènes de trouble, et outre une quantité d'un tensioactif inféricure à la quantité utilisée de G-CSF, au moins une substance tampon choisie dans l'ensemble comprenant l'acide acétique, l'acide lactique, l'acide citrique, l'acide maléique, l'acide phosphorique ou leurs sels ou l'arginine et ses sels, à une concentration finale de 2 à 100 mmol/l dans la solution pouvant être appliquée à l'état fini.

12. Utilisation d'acide acétique, d'acide lactique, d'acide citrique, d'acide maléique, d'acide phosphorique et de leurs sels, ou d'arginine et de ses sels, en tant que substances tampon pour éviter les phénomènes de trouble dans le cas de préparations pharmaceutiques aqueuses contenant du G-CSF, la concentration finale des substances tampon dans la solution pouvant être appliquée à l'état fini étant de 2 à 100 mmol/l, et les substances tampon étant utilisées en même temps qu'une quantité d'un tensioactif inférieure à la quantité utilisée de G-CSF.

13. Lyophilisat ou poudre préparés à partir d'une préparation selon l'une des revendications 1 à 9.

14. Préparation selon l'une des revendications 1 à 9, préparée par dissolution de lyophilisats ou de poudres selon la revendication 13 dans de l'eau ou dans des solutions aqueuses.
